(19) Europäisches Patentamt / European Patent Office / Office européen des brevets

(11) **EP 4 767 947 A1**

(12) **EUROPEAN PATENT APPLICATION**

(43) Date of publication:
01.07.2026 Bulletin 2026/27

(21) Application number: 25222654.3

(22) Date of filing: 11.12.2025

(51) International Patent Classification (IPC):
*A61B 6/06* (2006.01)     *A61B 6/00* (2024.01)

(52) Cooperative Patent Classification (CPC):
A61B 6/06; A61B 6/545

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 23.12.2024 KR 20240193779

(71) Applicant: **Synicsray**
Seoul, Jungnang-gu 02048 (KR)

(72) Inventors:
• **AN, Hyo-Tai**
12059 Namyangju-si (KR)
• **CHAE, Won Gi**
11902 Guri-si (KR)

(74) Representative: **Dragotti & Associati S.P.A.**
Via Nino Bixio, 7
20129 Milano (MI) (IT)

(54) **COLLIMATOR CONTROL APPARATUS AND METHOD USING ARTIFICIAL INTELLIGENCE-BASED IMAGE PROCESSING AND IMAGE ANALYSIS**

(57) The present disclosure relates to a collimator control apparatus and method using artificial intelligence-based image processing and image analysis. An apparatus according to an aspect of the present disclosure comprises: a 3D camera configured to acquire a depth image of an imaging target; an RGB camera configured to acquire an RGB image of the imaging target; a collimator configured to adjust an irradiation field; a memory storing at least one process associated with controlling the collimator based on the depth image and the RGB image; and a processor configured to execute the process. The processor inputs the depth image and the RGB image into an artificial intelligence model to output a depth map, recognizes an imaging region and an imaging angle using the depth map and the RGB image, extracts a contour of the recognized imaging region to define a measurement region, and sets the irradiation field of the collimator using the measurement region.

FIG. 1

EP 4 767 947 A1

**Description**

**CROSS-REFERENCE TO RELATED APPLICATION AND CLAIM OF**

**PRIORITY**

This application claims the benefit under 35 USC §119 of Korean Patent Application No. 10-2024-0193779, filed on December 23, 2024, in the Korean Intellectual Property Office, the entire disclosure of which is incorporated herein by reference for all purposes.

**BACKGROUND**

**1. FIELD**

The present disclosure relates to a collimator control apparatus and method using artificial intelligence-based image processing and image analysis. More particularly, the present disclosure relates to a collimator control apparatus and method for optimizing an irradiation field of diagnostic X-ray equipment and improving imaging quality using artificial intelligence-based image processing and image analysis utilizing RGB data and depth data.

**2. BACKGROUND ART**

Diagnostic X-ray equipment plays an important role in medical imaging, enabling non-invasive imaging of internal structures of humans or animals using radiation. Such equipment is designed to expose only desired regions to radiation using a collimator that adjusts an irradiation field; however, conventional X-ray equipment has several limitations in accurately setting or adjusting the irradiation field.

In conventional technology, a method of manually setting the angle of a collimator has been mainly used, which makes it difficult to predict the exact size and shape of an imaging region, causes variations depending on user proficiency, and requires significant time, thereby delaying imaging time. If the irradiation field is set too narrow, only a portion of an imaging target is included, necessitating re-imaging, and conversely, if set too wide, patient safety is compromised due to unnecessary radiation exposure. Additionally, conventional systems often fail to accurately detect the contour of an imaging region, causing radiation to deviate from necessary regions or include only insufficient areas, resulting in degraded image quality and missing diagnostic information.

As a result, cases frequently occur where images are clipped or the irradiation field is inaccurate, requiring re-imaging, which demands additional time and effort from both patients and medical staff. Furthermore, large hospitals or health screening specialized hospitals frequently use collimators fully opened without adjustment to diagnose many patients within a short time, which may cause patients to be exposed to more radiation than appropriate. Therefore, there is an increasing need for technology to precisely recognize imaging regions of diagnostic X-ray equipment and automatically adjust the angle of collimators for rapid diagnosis and prevention of patient exposure.

(Patent Document 1) Korean Patent Publication No. 10-2604560

**SUMMARY**

An embodiment disclosed in the present disclosure aims to accurately recognize an imaging region and an imaging angle and automatically set an irradiation field, thereby preventing radiation exposure errors caused by manual operation and improving imaging efficiency.

Additionally, an embodiment disclosed in the present disclosure aims to precisely extract contours based on RGB and depth data and optimize the irradiation field by reflecting an appropriate margin, thereby reducing unnecessary radiation exposure and minimizing patient radiation dose.

Additionally, an embodiment disclosed in the present disclosure aims to determine whether an image is clipped using quantitative evaluation criteria such as a clipping coefficient, and automatically correct and re-image when the irradiation field is not appropriate, thereby improving image quality.

Additionally, an embodiment disclosed in the present disclosure aims to set an irradiation field that accurately reflects subject characteristics through integrated analysis of RGB and depth data, and improve image quality and diagnostic accuracy through region-specific corrections.

Additionally, an embodiment disclosed in the present disclosure aims to quantitatively evaluate the opening degree of a collimator and optimize collimator settings based on an exposure area ratio and a distance index, thereby maintaining appropriateness of the irradiation field.

According to one aspect of the present disclosure, an apparatus comprises: a 3D camera configured to acquire a depth image of an imaging target; an RGB camera configured to acquire an RGB image of the imaging target; a collimator configured to adjust an irradiation field; a memory storing at least one process associated with an operation for controlling the collimator based on the depth image and the RGB image; and a processor configured to execute the operation according to the process. The processor is configured to input the depth image and the RGB image into an artificial intelligence model to output a depth map, recognize an imaging region and an imaging angle using the depth map and the RGB image, extract a contour of the recognized imaging region to define a measurement region, and set the irradiation field of the collimator using the measurement region.

According to another aspect of the present disclosure, a method is performed by an apparatus comprising: a 3D camera configured to acquire a depth image of an imaging target; an RGB camera configured to acquire an RGB image of the imaging target; a collimator configured to adjust an irradiation field; a memory storing at least one process associated with an operation for controlling the collimator based on the depth image and the RGB image; and a processor configured to execute the operation according to the process. The method comprises: inputting, by the processor, the depth image and the RGB image into an artificial intelligence model to output a depth map; recognizing, by the processor, an imaging region and an imaging angle using the depth map and the RGB image; extracting, by the processor, a contour of the recognized imaging region to define a measurement region; and setting, by the processor, the irradiation field of the collimator using the measurement region.

## BRIEF DESCRIPTION OF THE DRAWINGS

FIG. 1 is a block diagram schematically illustrating a collimator control apparatus according to an example of the present disclosure.

FIG. 2 is a flowchart illustrating a collimator control method according to an example of the present disclosure.

FIG. 3 is a flowchart illustrating a method of outputting a depth map according to an example of the present disclosure.

FIG. 4 is a flowchart illustrating a method of setting an irradiation field according to an example of the present disclosure.

FIGS. 5, 6(a), 6(b), and 6(c) are exemplary diagrams illustrating a method of extracting a contour according to an example of the present disclosure.

FIG. 7 is a flowchart illustrating a method of determining whether an image is clipped according to an example of the present disclosure.

FIG. 8 is a flowchart illustrating a method of determining an opening degree of a collimator according to an example of the present disclosure.

FIGS. 9 and 10 are exemplary diagrams illustrating a method of determining an opening degree of a collimator according to an example of the present disclosure.

## DETAILED DESCRIPTION OF THE EMBODIMENTS

The same reference numerals refer to the same components throughout the present disclosure. The present disclosure does not describe all elements of the embodiments, and general content in the technical field to which the present disclosure belongs or content overlapping between embodiments is omitted. The terms "unit, module, member, block" used in the specification may be implemented as software or hardware, and depending on embodiments, a plurality of "units, modules, members, blocks" may be implemented as a single component, or a single "unit, module, member, block" may include a plurality of components.

Throughout the specification, when a part is said to be "connected" to another part, this includes not only cases where the part is directly connected but also cases where the part is indirectly connected, and indirect connection includes connection through a wireless communication network.

Additionally, when a part is said to "include" a component, this means that the part may further include other components, not excluding other components, unless specifically stated otherwise.

Throughout the specification, when a member is said to be positioned "on" another member, this includes not only cases where the member is in contact with the other member but also cases where another member exists between the two members.

Terms such as first and second are used to distinguish one component from another component, and components are not limited by these terms.

Singular expressions include plural expressions unless the context clearly indicates otherwise.

Identification codes in each step are used for convenience of explanation and do not describe the order of each step, and each step may be performed differently from the stated order unless a specific order is clearly stated in the context.

In this specification, "an apparatus according to the present disclosure" includes all various apparatuses capable of performing computational processing and providing results to a user. For example, an apparatus according to the present disclosure may include or be any one of a computer, a server apparatus, diagnostic X-ray equipment, a hardware apparatus installed inside X-ray equipment, and a portable terminal.

Hereinafter, embodiments of the present disclosure will be described in detail with reference to the accompanying drawings.

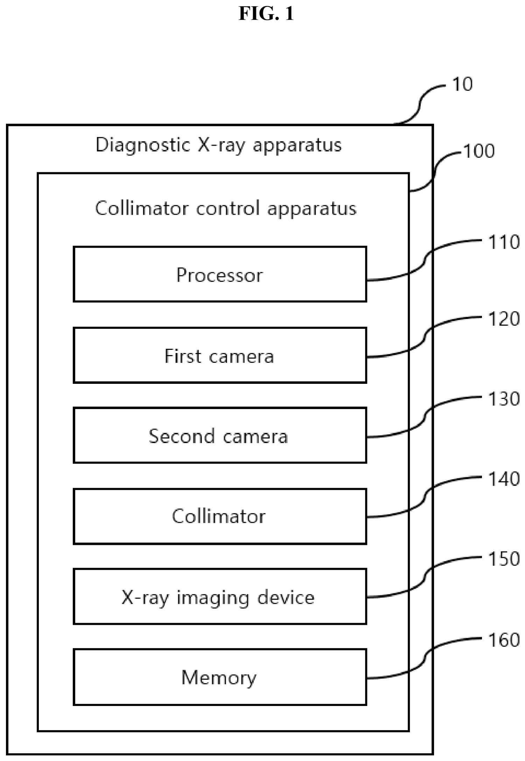

FIG. 1 is a block diagram schematically illustrating a collimator control apparatus according to an example of the present disclosure. The components illustrated in FIG. 1 are not essential for implementing an apparatus 100 according to the present disclosure, and the apparatus 100 described in this specification may have more or fewer components than those listed above.

Referring to FIG. 1, a collimator control apparatus 100 according to an embodiment of the present disclosure may be included as a component of a diagnostic X-ray apparatus 10, and may include a processor 110, a first camera which is a 3D camera 120, a second camera which is an RGB camera 130, a collimator 140, an X-ray imaging device 150, and a memory 160. The collimator control apparatus 100 may automatically adjust an irradiation field, generate high-quality images, and minimize patient radiation dose.

In one embodiment, the processor 110 may perform operations of processing input images to set and optimize an irradiation field. The processor 110 may receive depth data and RGB data acquired from the first camera and the second camera and generate a depth map through an artificial intelligence model (CNN). The processor 110 may fusion-analyze the generated depth map and RGB image to precisely extract the contour and measurement region of an imaging target, and based on this, set the irradiation field of the collimator 140. Additionally, the processor 110 may analyze captured images to determine whether the image is clipped and whether the irradiation field is appropriate, and automatically correct the irradiation field and perform re-imaging when necessary.

In one embodiment, the first camera, which is a 3D camera 120, may acquire depth data of an imaging target, which provides information including the three-dimensional structure and thickness of the target. The depth data may be utilized to analyze the volume and shape of an imaging target and precisely set an irradiation field.

In one embodiment, the second camera, which is an RGB camera 130, may acquire an image including appearance and color data of an imaging target. The RGB image is useful for contour extraction and imaging angle analysis, and may be fused with depth data from the 3D camera 120 to increase accuracy.

In one embodiment, the collimator 140 is an apparatus that may limit or expand the range through which radiation passes according to control commands from the processor 110. The collimator 140 sets an irradiation field based on the measurement region and margin calculated by the processor 110, and may prevent radiation from spreading to unnecessary areas. This may reduce radiation dose and improve image quality.

In one embodiment, the X-ray imaging device 150 may acquire an image of an imaging target using an irradiation field adjusted through the collimator 140. The captured image is analyzed by the processor 110, and if the image is clipped or quality does not meet standards, the irradiation field may be readjusted and re-imaging may be performed.

In one embodiment, the memory 160 stores data related to operations of the processor 110, and may include an artificial intelligence model and irradiation field setting data. The memory 160 may provide information needed when the processor 110 performs image analysis and collimator control operations.

In one embodiment, the present disclosure may integrally analyze data acquired from the first camera, which is a 3D camera 120, and the second camera, which is an RGB camera 130, in the processor 110 to automatically adjust and optimize an irradiation field. In particular, the present disclosure may fuse depth data and RGB data to precisely extract the contour of an imaging target, analyze an imaging region and angle, and set an irradiation field to irradiate only necessary areas based on this. Through this, diagnostic X-ray equipment may provide high-quality images, reduce radiation dose, and improve safety for patients and medical staff.

Meanwhile, the collimator control apparatus 100 of the present disclosure may be integrated and designed inside diagnostic X-ray equipment to maintain device integrity and may be installed in a compact size. The apparatus 100 is protected by a radiation shielding cover to prevent radiation leakage and may be fixedly installed in an appropriate location inside the equipment depending on the usage environment. The X-ray imaging device 150 may comprise an X-ray tube and a detector. The collimator 140 is positioned between the X-ray tube and the detector and may include variable blades made of high-density metal. The blades move through a precision motor and are adjusted so that radiation is accurately irradiated only to target areas according to the irradiation field set by the processor. The two cameras are installed inside the X-ray equipment facing the imaging target, and may be designed with a robust radiation shielding structure to prevent external interference during operation.

Meanwhile, the processor 110 may be implemented with a memory storing an algorithm or data for a program reproducing the algorithm for controlling operations of components within the apparatus, and at least one processor (not shown) performing the aforementioned operations using data stored in the memory. At this time, the memory and the processor may each be implemented as separate chips. Alternatively, the memory and the processor may be implemented as a single chip.

Meanwhile, at least one component may be added or deleted corresponding to the performance of the components illustrated in FIG. 1. Additionally, it will be easily understood by those skilled in the art that the mutual positions of the components may be changed corresponding to the performance or structure of the system.

Meanwhile, the processor 110 refers to software and/or hardware components such as Field Programmable Gate Array (FPGA) and Application Specific Integrated Circuit (ASIC).

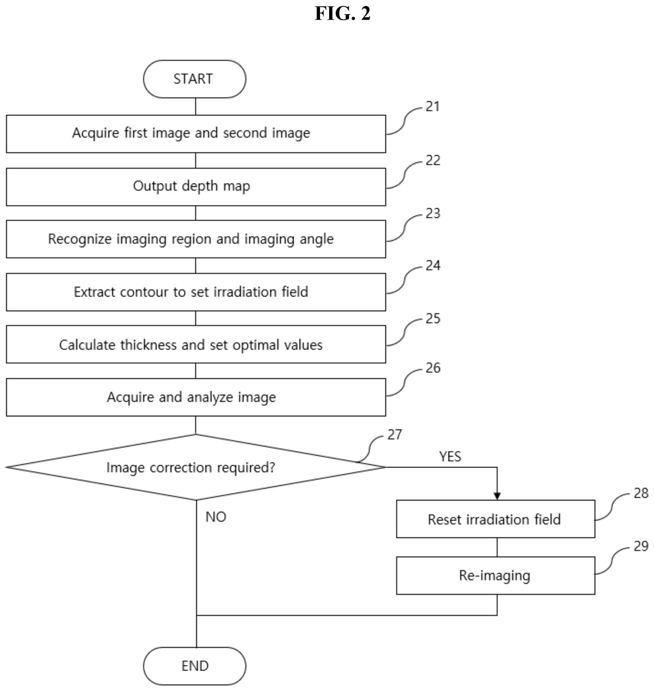

FIG. 2 is a flowchart illustrating a collimator control method according to an example of the present disclosure.

Referring to FIG. 2, in one embodiment, in operation 21, the first camera and the second camera may acquire a first image and a second image of an imaging target, respectively. Here, the first camera is a 3D camera 120 that may provide a depth image including depth data, and the second camera is an RGB camera 130 that may provide an RGB image including color and appearance information.

In one embodiment, in operation 22, the processor 110 may input the first image and the second image into an artificial intelligence model to generate a depth map. The learning model used at this time is an artificial intelligence algorithm such as CNN (Convolutional Neural Network) that may precisely analyze the three-dimensional shape and structure of an imaging target based on data provided from the first camera and the second camera. The generated depth map may be used for data analysis in subsequent steps and for setting an irradiation field together with the RGB image.

In one embodiment, in operation 23, the processor 110 may recognize an imaging region and an imaging angle using the depth map and the RGB image. In this process, the appearance of an imaging target is analyzed based on visual information of the RGB image, and the depth map may additionally supplement the thickness and three-dimensional structure of the imaging target. Through this data, the processor 110 may precisely calculate the region and angle of the imaging target.

In one embodiment, in operation 24, the processor 110 may extract a contour of an imaging region to define a measurement region, and set an irradiation field of the collimator 140 based on this measurement region. Contour extraction is performed through combined analysis of the RGB image and the depth map, and filtering and edge detection algorithms may be used. The defined measurement region may be adjusted to include a margin of 1 to 2 cm as needed so that the irradiation field includes the entire region.

In one embodiment, in operation 25, the processor 110 may calculate the thickness of an imaging target and set an optimal radiation dose value based on this. This process calculates the radiation intensity needed for radiation to completely penetrate the subject and may prevent unnecessary radiation exposure.

In one embodiment, in operation 26, the processor 110 may acquire an image using the irradiation field through the X-ray imaging device 150 and analyze the captured image. During the image analysis process, the processor 110 may evaluate image quality and determine whether the image is clipped or the irradiation field is inappropriate.

In one embodiment, in operation 27, the processor 110 may determine whether the captured image is clipped. This determination may be made based on luminance differences of the captured image, and may evaluate whether the image is clipped by analyzing brightness value differences between regions. Additionally, the processor 110 may calculate a ratio between the irradiation field and a detector area of the captured image to determine an opening degree of the collimator 140.

In one embodiment, in operation 28, when the processor 110 determines that image correction is needed, it may reset the irradiation field based on analysis results. Resetting the irradiation field may be done by resolving clipping problems or adjusting the opening degree of the irradiation field.

In one embodiment, in operation 29, a re-imaging step may be performed in which the imaging target is re-imaged based on the reset irradiation field. This process optimizes the irradiation field to ensure that the entire area of the imaging target is included, and may ultimately generate high-quality images.

In one embodiment, alternatively, in operation 28, when image correction is not needed, the processor 110 may end imaging.

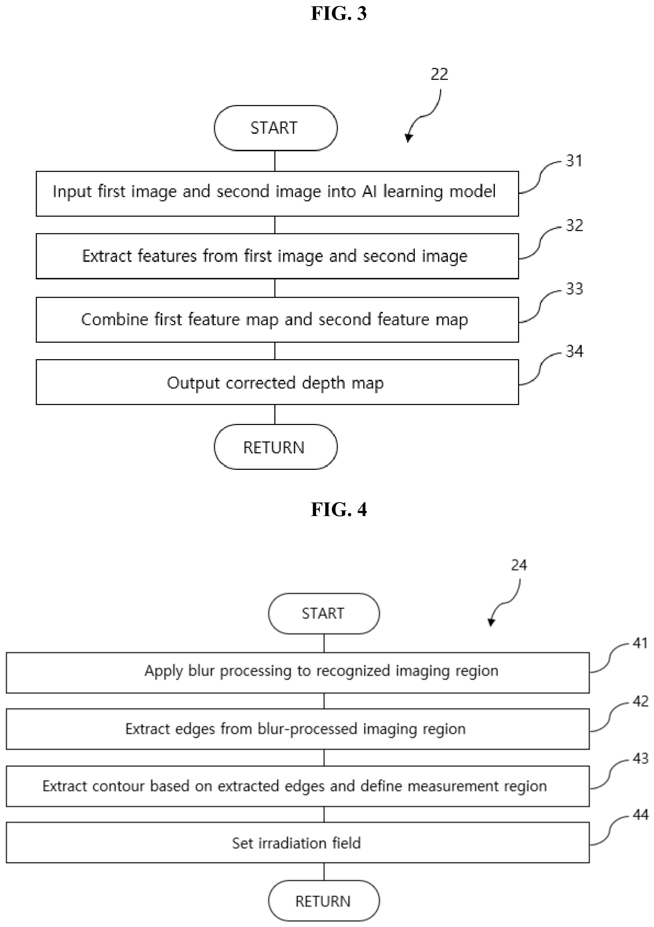

FIG. 3 is a flowchart illustrating a method of outputting a depth map according to an example of the present disclosure. FIG. 3 is a flowchart showing detailed operations of operation 22 of FIG. 2.

Referring to FIG. 3, in one embodiment, in operation 31, the processor 110 may input a first image and a second image into an artificial intelligence model. The first image is a depth image acquired from the 3D camera 120, and the second image may be an RGB image acquired from the RGB camera 130. The depth image includes distance and thickness information between the target and the camera, and the RGB image provides color and appearance information of the imaging target. These two images may be used as data for comprehensively analyzing three-dimensional information and appearance of the imaging target. The learning model is configured based on CNN (Convolutional Neural Network) and may extract main features of the imaging target through input data and contribute to improving the accuracy of depth data.

In one embodiment, in operation 32, the processor 110 may extract feature data from the first image and the second image. Here, the feature data includes unique structural, color, and spatial information of the imaging target from each image. The depth image is data representing distance and thickness of the imaging target and may be utilized as basic data for three-dimensional analysis. The RGB image provides color and appearance information of the imaging target and may be used to clearly define boundaries of the target. In this process, CNN layers may analyze input images and identify meaningful features from each image to generate feature maps.

In one embodiment, in operation 33, the processor 110 may combine the first feature map and the second feature map. The two feature maps may complementarily integrate RGB data and depth data to express information about the imaging target more richly. This combination process includes RGB-D data fusion, and machine learning-based loss functions may be used to reduce differences between the two data and maintain consistency of fusion. The loss function is designed to minimize differences between the corrected depth map and the actual depth map, so that the combined feature map may more accurately reflect the actual structure of the imaging target.

Additionally, the present disclosure may consider technologies such as triangulation-based correction or Optical Flow to correct errors in depth images based on RGB data provided from the RGB camera 130, but in the present embodiment, a correction process is performed using a CNN-based machine learning model. At this time, an RGB image (I_RGB) and a noisy depth image (D_noisy) are used as input values, and feature maps extracted from each image are combined through CNN layers. Through the combined feature data, a refined depth map (D_refined) may be output, and this process may be expressed by the following equation.

$$D\_refined = f\_CNN(I\_RGB, D\_noisy)$$

Here, D_refined represents the refined depth map, I_RGB is data acquired from the RGB camera, and D_noisy means noisy depth data. f_CNN represents a CNN-based correction function and may derive optimal correction results by minimizing differences between input values.

In one embodiment, in operation 34, the processor 110 may output the refined depth map. The refined depth map is generated by fusing the RGB image and the depth image, and may precisely represent the three-dimensional structure and appearance of the imaging target. This map may be used as main input data for extracting contours of the imaging target or defining measurement regions in subsequent steps, and accurately adjusting the irradiation field of the collimator. The refined depth map particularly plays an essential role in automatic setting and optimization of the irradiation field, preventing unnecessary radiation exposure and ensuring that only the imaging target area is accurately irradiated.

After completing operation 34, the processor 110 may proceed to operation 23.

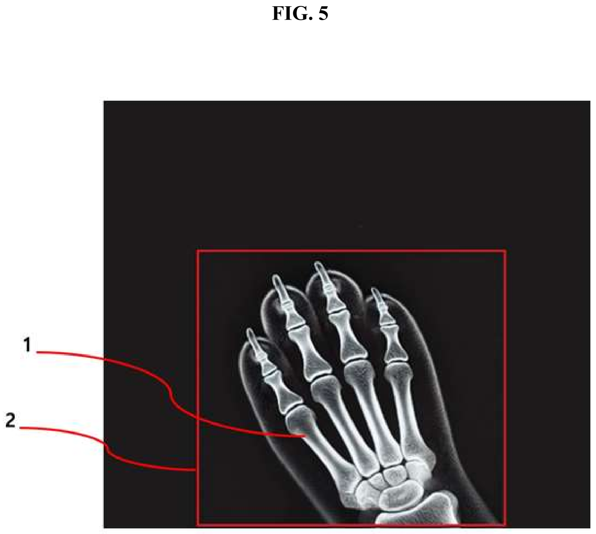

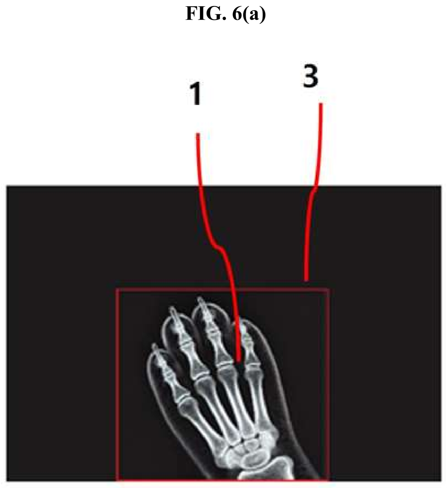

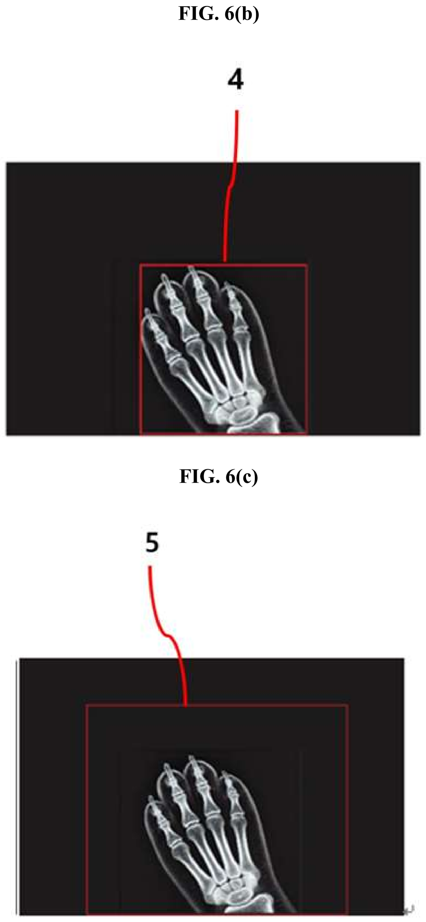

FIG. 4 is a flowchart illustrating a method of setting an irradiation field according to an example of the present disclosure. FIGS. 5, 6(a), 6(b), and 6(c) are exemplary diagrams illustrating a method of extracting a contour according to an example of the present disclosure. FIG. 4 is a flowchart showing detailed operations of operation 24 of FIG. 2.

Referring to FIGS. 4, 5, 6(a), 6(b), 6(c), in one embodiment, in operation 41, the processor 110 may blur-process the recognized imaging region. Blur processing is performed to remove unnecessary details of the imaging region and emphasize the basic shape for contour extraction. In this process, various blur processing techniques such as Gaussian filter, median filter, or bilateral filter may be used. For example, a Gaussian filter smoothly processes image noise, and a bilateral filter is useful for reducing noise while preserving edge information. When an imaging region has a complex structure, blur processing may remove unnecessary details and improve the accuracy of edge detection performed in subsequent steps.

In one embodiment, in operation 42, the processor 110 may extract edges from the blur-processed imaging region. Edge detection is a step of emphasizing boundaries of an imaging target, and Sobel, Prewitt, Laplacian of Gaussian (LoG), or Canny edge detection algorithms may be used. In this process, points of rapid change in pixel values may be identified to clearly reveal boundaries of the imaging target. For example, a Canny edge detection algorithm provides the most suitable edge information through a multi-stage process and may be used to generate an initial contour 2 surrounding an imaging region 1 illustrated in FIG. 5.

In one embodiment, in operation 43, the processor 110 may form a contour based on the extracted edges and define a measurement region based on this. Contour extraction is a step for clearly distinguishing the boundary and internal structure of an imaging target, and may be implemented through image processing libraries such as OpenCV. In this process, when a contour 3 with an appropriate range is extracted as illustrated in FIG. 6(a), the contour extraction process may be terminated. However, when a contour 4 is too narrow as in FIG. 6(b) or a contour 5 is too wide as in FIG. 6(c), the processor may recalculate the contour to define an appropriate measurement region. Through this iterative process, the accuracy of contours and measurement regions is ensured, and an optimal irradiation field may be set.

In one embodiment, in operation 44, the processor 110 may set an irradiation field by adding a predefined spatial margin to the defined measurement region. This spatial margin provides allowance so that the irradiation field may completely cover the entire imaging target, and an additional margin of 1 to 2 cm is generally set. This process may help the collimator 140 physically limit or expand the irradiation field to implement an accurate range. For example, if the collimator sets the irradiation field too narrow, part of the imaging target may be omitted, and conversely, if set too wide, radiation dose may increase due to unnecessary radiation exposure. To prevent these problems, an appropriate spatial margin is set, and the collimator may physically adjust the irradiation field based on this.

After completing operation 44, the processor 110 may proceed to operation 25.

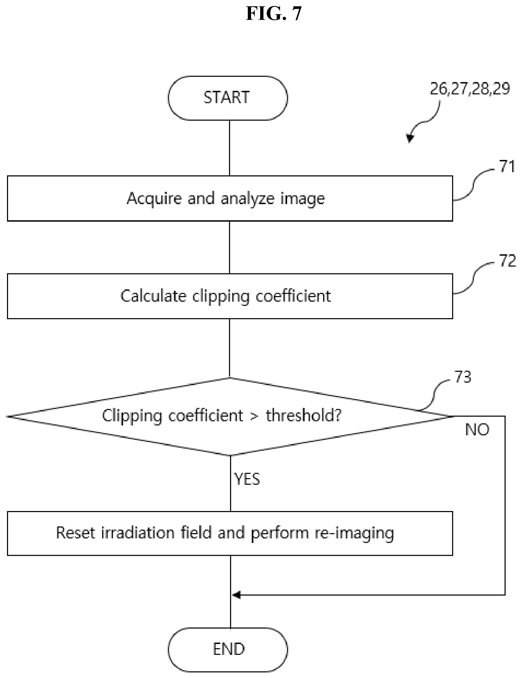

FIG. 7 is a flowchart illustrating a method of determining whether an image is clipped according to an example of the present disclosure. FIG. 7 is a flowchart showing detailed operations of operations 26, 27, 28, and 29 of FIG. 2.

Referring to FIG. 7, in one embodiment, in operation 71, the processor 110 may capture and analyze an image of an imaging region. In this process, the processor 110 receives an image captured through an X-ray imaging device as input to begin analysis and evaluates whether the captured image is appropriately included within the irradiation field. This evaluation process includes operations of analyzing factors such as contrast ratio, luminance distribution, and luminance differences between pixels of the imaging target. In particular, the quality and consistency of the image may be precisely evaluated by calculating an average luminance value of outermost single-pixel regions.

In one embodiment, in operation 72, the processor 110 may calculate a clipping coefficient (C) based on image analysis results. The clipping coefficient is used as an indicator to quantitatively evaluate whether a captured image is clipped and may be calculated according to Equation 1 below.

[Equation 1]

$$C = w\_left\text{-}right \times \Delta L\_left\text{-}right + w\_top\text{-}bottom \times \Delta L\_top\text{-}bottom$$

Here, $\Delta L\_left\text{-}right$ represents the left-right luminance difference, and $\Delta L\_top\text{-}bottom$ represents the top-bottom luminance difference. $w\_left\text{-}right$ is a weight applied to the left-right luminance difference, and $w\_top\text{-}bottom$ is a weight applied to the top-bottom luminance difference.

For example, the left-right luminance difference may be calculated as follows:

$$\Delta L\_left\text{-}right = |\, \Delta L\_left - \Delta L\_right\, |$$

The top-bottom luminance difference may be calculated as follows:

$$\Delta L\_top\text{-}bottom = |\, \Delta L\_top - \Delta L\_bottom\, |$$

In one embodiment, the clipping coefficient is calculated based on brightness differences occurring in outermost single-pixel regions and quantitatively evaluates whether an image is clipped. Through this calculation, the processor 110 may determine whether the imaging target is appropriately included within the irradiation field.

In one embodiment, in operation 73, the processor 110 may determine whether the clipping coefficient exceeds a preset threshold. The threshold is preset as a criterion for determining whether the imaging target is appropriately included within the irradiation field. The processor 110 may compare the clipping coefficient with the threshold to determine whether the image is clipped. For example, if the calculated clipping coefficient exceeds the threshold, it may be interpreted that the irradiation field did not completely cover the imaging target.

In one embodiment, in operation 74, when the clipping coefficient exceeds the threshold and the image is determined to be clipped, the processor 110 may reset the irradiation field and perform re-imaging. Resetting the irradiation field includes operations of readjusting the measurement region based on the contour of the imaging target, and adjusting the collimator 140 based on this to expand or reduce the irradiation field. In this process, the irradiation field may be adjusted to completely cover the imaging target through contour re-extraction and measurement region resetting. Re-imaging is performed by applying the modified irradiation field, and accurate imaging data may be acquired through this.

Meanwhile, in operation 73, when the clipping coefficient does not exceed the threshold, the processor 110 may determine that the irradiation field is appropriately set and end imaging without additional adjustment. In this case, the captured image is considered to meet the quality standards required in the analysis step, and may proceed to the next step or end imaging.

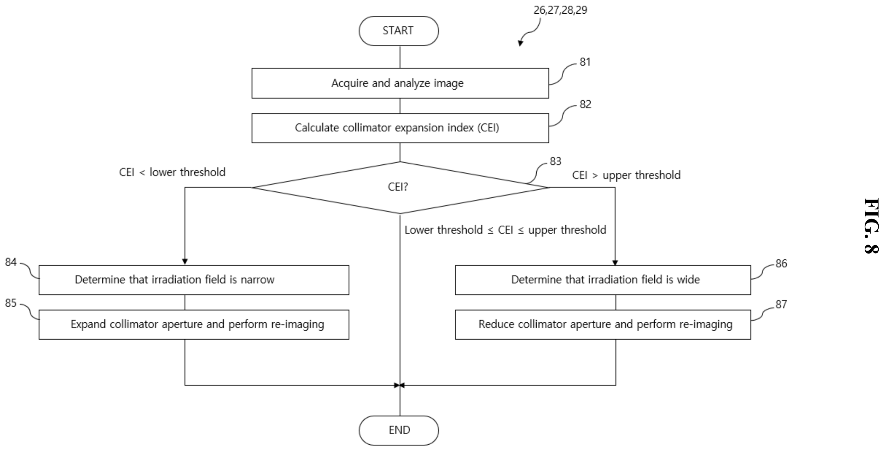

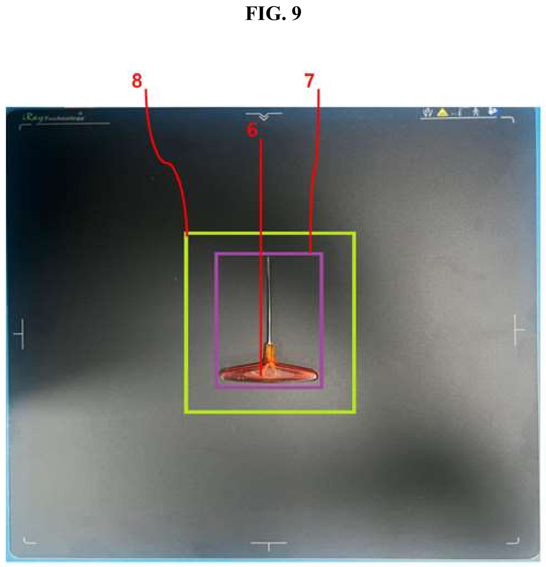

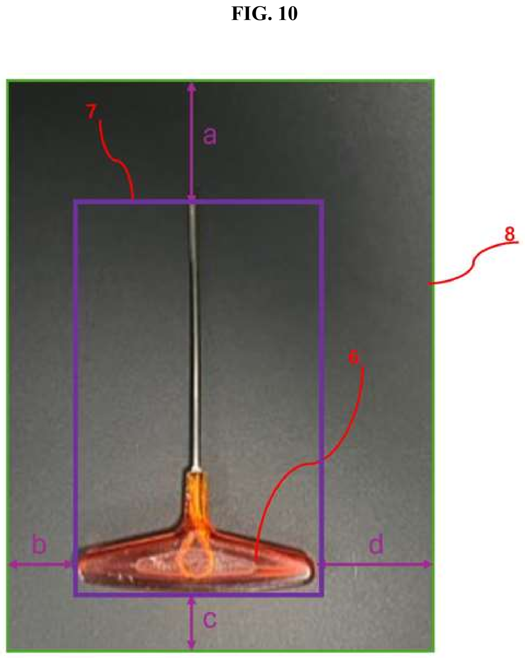

FIG. 8 is a flowchart illustrating a method of determining an opening degree of a collimator according to an example of the present disclosure. FIGS. 9 and 10 are exemplary diagrams illustrating a method of determining an opening degree of a collimator according to an example of the present disclosure. FIG. 8 is a flowchart showing detailed operations of operations 26, 27, 28, and 29 of FIG. 2.

Referring to FIGS. 8 to 10, in one embodiment, in operation 81, the processor 110 may capture and analyze an image of an imaging region. In this process, an image captured using the X-ray imaging device includes an X-ray exposed area 7 within a detector area 8. As illustrated in FIG. 9, the X-ray exposed area 7 located at the center of an imaging target 6 is surrounded by the detector area 8. The detector area 8 represents the entire area irradiated with radiation, and the X-ray exposed area 7 represents the area irradiated to the imaging target region. In this step, the captured image is digitized and input to the processor 110, and may be used as basic data for evaluating whether the imaging target is appropriately included within the irradiation field.

In one embodiment, in operation 82, the processor 110 may calculate a collimator expansion index (CEI) based on image analysis results. The CEI may be calculated according to Equation 2 below:

[Equation 2]

$$CEI = w_1 \times ER + w_2 \times DI$$

Here, ER represents an exposure area ratio, and DI represents a distance index. $w_1$ is a weight for ER, and $w_2$ represents a weight for DI.

The exposure area ratio (ER) is calculated according to Equation 3 below.

[Equation 3]

$$ER = A\_exposed / A\_detector$$

Here, A_exposed is the X-ray exposed area 7, and A_detector is the detector area 8. The X-ray exposed area 7 may be analyzed through an X-ray histogram, and among values of 0 to 65535, a threshold may be manually set or set using histogram analysis or Otsu algorithm, and the exposure area may be calculated using the set threshold. As shown in FIG. 9, the X-ray exposed area 7 is displayed in purple, and the detector area 8 is displayed in green. An ER value may be derived by calculating the number of pixels in these two areas.

The distance index (DI) is calculated using top, bottom, left, and right distances a, b, c, d between the X-ray exposed area 7 and the detector area 8 as illustrated in FIG. 10. For example, if the value of (a + b + c + d) is less than 0.5 cm, a negative sign is attached to the absolute value of the corresponding difference; if the value of (a + b + c + d) is greater than 1.5 cm, the absolute value of the corresponding difference is displayed. DI quantitatively evaluates the distance difference between the irradiation field and the imaging target, and may identify when the distance size exceeds or falls below an ideally set value.

In one embodiment, in operation 83, the processor 110 may compare the CEI with preset lower and upper thresholds. If the CEI is less than the lower threshold, it may be determined that the irradiation field is narrow compared to the imaging target. If the CEI exceeds the upper threshold, it may be determined that the irradiation field is excessively wide. On the other hand, if the CEI is greater than or equal to the lower threshold and less than or equal to the upper threshold, the irradiation field may be considered to be appropriately set.

In one embodiment, in operations 84 and 85, when the CEI is less than the lower threshold, the processor 110 may determine that the irradiation field is narrow, expand an aperture of the collimator, and perform a re-imaging step with a new irradiation field. When the distance between the X-ray exposed area 7 and the detector area 8 in FIG. 10 is too narrow, this means that the irradiation field did not sufficiently cover the imaging target. The processor 110 may expand the aperture of the collimator based on the contour of the X-ray exposed area and acquire accurate data through re-imaging to resolve this problem.

In one embodiment, in operations 86 and 87, when the CEI exceeds the upper threshold, the processor 110 may determine that the irradiation field is wide, reduce the aperture of the collimator, and perform re-imaging. When the X-ray exposed area 7 exceeds the boundary of the detector area 8 or unnecessarily wide radiation exposure occurs, the processor 110 may reduce the aperture of the collimator to maintain an appropriate irradiation field. Through this process, radiation exposure may be optimized and unnecessary radiation dose may be minimized.

In one embodiment, as an additional correction process, the collimator control apparatus 100 according to the present disclosure may set an irradiation field that accurately reflects characteristics of an imaging target based on RGB images and depth images, thereby improving imaging quality and minimizing radiation exposure. For example, the apparatus 100 may compare RGB images and depth images, evaluate how well each data reflects specific characteristics of an imaging target, and then dynamically adjust weights to set an optimal irradiation field.

For example, if an RGB image more clearly represents a contour in a specific region, the processor 110 may increase the weight of the RGB image, and if a depth image better reflects the depth or structural characteristics of a subject, it may increase the weight of the depth image. Through this, the irradiation field may be adjusted to accurately reflect the actual characteristics of the imaging target.

Additionally, when errors are identified by analyzing X-ray images, the apparatus 100 may readjust the aperture of the collimator based on correction values set for each region. Main indicators used in this correction process include a clipping coefficient (C) and a collimator expansion index (CEI). These two indicators may be determined according to weight ratios of RGB and Depth images, respectively.

For example, when imaging a chest X-ray, if an RGB image better reflects subject characteristics, correction is performed by increasing the weight of the RGB image, and if a depth image provides more accurate information, correction is performed by increasing the weight of the Depth image. Through this, an irradiation field appropriate for region-specific characteristics may be set.

When proceeding with re-imaging after detecting errors in an X-ray image, the apparatus 100 may adjust the aperture of the collimator based on preset correction values for each region. The correction values used at this time may reflect result values calculated according to weight ratios of RGB images and Depth images. For example, when an RGB image more clearly reflects the contour of a subject, the contribution of the RGB image is increased, and when a depth image better reflects the depth or internal structural characteristics of a subject, the contribution of the depth image may be increased. Such weight adjustments may be automatically performed through machine learning algorithms and may be trained to preferentially utilize images that effectively reflect specific characteristics of a subject.

Through this, the collimator control apparatus according to the present disclosure may increase the accuracy of the irradiation field and significantly improve the quality of captured images. This may minimize unnecessary radiation exposure, ensure safety for both patients and medical staff, and improve diagnostic accuracy.

Meanwhile, the disclosed embodiments may be implemented in the form of a recording medium storing instructions executable by a computer. Instructions may be stored in the form of program code and, when executed by a processor, may generate a program module to perform operations of the disclosed embodiments. The recording medium may be implemented as a computer-readable recording medium.

Computer-readable recording media include all types of recording media storing instructions that may be interpreted by a computer. For example, these may include ROM (Read Only Memory), RAM (Random Access Memory), magnetic tape, magnetic disk, flash memory, optical data storage devices, and the like.

As described above, the disclosed embodiments have been described with reference to the accompanying drawings. Those skilled in the art to which the present disclosure belongs will understand that the present disclosure may be practiced in forms different from the disclosed embodiments without changing the technical spirit or essential features of the present disclosure. The disclosed embodiments are illustrative and should not be construed as limiting.

**Claims**

1.    A collimator control apparatus (100) comprising:

   a 3D camera (120) configured to acquire a depth image of an imaging target;
   an RGB camera (130) configured to acquire an RGB image of the imaging target;
   a collimator (140) configured to adjust an irradiation field;
   a memory (160) storing at least one process associated with an operation for controlling the collimator (140) based on the depth image and the RGB image; and
   a processor (110) configured to execute the operation according to the process,
   wherein the processor (110) is configured to:

      input the depth image and the RGB image into an artificial intelligence model to output a depth map;
      recognize an imaging region and an imaging angle using the depth map and the RGB image;
      extract a contour of the recognized imaging region to define a measurement region; and
      set the irradiation field of the collimator (140) using the measurement region.

2.    The collimator control apparatus (100) of claim 1, further comprising an X-ray imaging device (150) configured to acquire an X-ray image of the imaging region,
   wherein the processor (110) is configured to acquire the X-ray image using the irradiation field through the X-ray imaging device (150), and determine whether the X-ray image is clipped using a luminance difference between regions of the X-ray image.

3. The collimator control apparatus (100) of claim 2, wherein the processor (110) is configured to:

calculate a clipping coefficient (C) according to Equation 1 below; and
determine that the X-ray image is clipped when the clipping coefficient (C) exceeds a preset threshold.

[Equation 1]

$$C = w\_left\text{-}right \times \Delta L\_left\text{-}right + w\_top\text{-}bottom \times \Delta L\_top\text{-}bottom$$

wherein C is a clipping coefficient, $\Delta L\_left\text{-}right$ is a left-right luminance difference, $\Delta L\_top\text{-}bottom$ is a top-bottom luminance difference, $w\_left\text{-}right$ is a weight applied to the left-right luminance difference, and $w\_top\text{-}bottom$ is a weight applied to the top-bottom luminance difference.

4. The collimator control apparatus (100) of claim 3, wherein the processor (110) is configured to determine an opening degree of the collimator (140) using a ratio and a distance between an X-ray exposed area (7) and a detector area (8).

5. The collimator control apparatus (100) of claim 4, wherein the processor (110) is configured to:

calculate a collimator expansion index (CEI) according to Equation 2 below;
determine that the irradiation field is narrow when the collimator expansion index (CEI) is less than a lower threshold; and
determine that the irradiation field is wide when the collimator expansion index (CEI) exceeds an upper threshold.

[Equation 2]

$$CEI = w_1 \times ER + w_2 \times DI$$

wherein CEI is a collimator expansion index, ER is an exposure area ratio, DI is a distance index, $w_1$ is a weight for ER, and $w_2$ is a weight for DI.

6. The collimator control apparatus (100) of claim 5, wherein the processor (110) is configured to:

expand an aperture of the collimator (140) in a re-imaging step when the irradiation field is determined to be narrow; and
reduce the aperture of the collimator (140) in the re-imaging step when the irradiation field is determined to be wide.

7. The collimator control apparatus (100) of claim 6, wherein the processor (110) is configured to optimize the irradiation field by preferentially utilizing an image that more accurately reflects characteristics of the imaging target between the depth image and the RGB image.

8. A collimator control method performed by an apparatus (100) comprising:

a 3D camera (120) configured to acquire a depth image of an imaging target;
an RGB camera (130) configured to acquire an RGB image of the imaging target;
a collimator (140) configured to adjust an irradiation field;
a memory (160) storing at least one process associated with an operation for controlling the collimator (140) based on the depth image and the RGB image; and
a processor (110) configured to execute the operation according to the process,
the method comprising:

inputting, by the processor (110), the depth image and the RGB image into an artificial intelligence model to output a depth map;
recognizing, by the processor (110), an imaging region and an imaging angle using the depth map and the RGB image;
extracting, by the processor (110), a contour of the recognized imaging region to define a measurement region; and

setting, by the processor (110), the irradiation field of the collimator (140) using the measurement region.

**FIG. 1**

Diagnostic X-ray apparatus — 10

Collimator control apparatus — 100

| Processor | 110 |
| First camera | 120 |
| Second camera | 130 |
| Collimator | 140 |
| X-ray imaging device | 150 |
| Memory | 160 |

**FIG. 2**

**FIG. 3**

22

START

| Input first image and second image into AI learning model | 31 |

| Extract features from first image and second image | 32 |

| Combine first feature map and second feature map | 33 |

| Output corrected depth map | 34 |

RETURN

**FIG. 4**

24

START

| Apply blur processing to recognized imaging region | 41 |

| Extract edges from blur-processed imaging region | 42 |

| Extract contour based on extracted edges and define measurement region | 43 |

| Set irradiation field | 44 |

RETURN

**FIG. 5**

**FIG. 6(a)**

**FIG. 6(b)**

4

**FIG. 6(c)**

5

**FIG. 7**

**FIG. 8**

**FIG. 9**

**FIG. 10**

# EP 4 767 947 A1

Europäisches
Patentamt
European
Patent Office
Office européen
des brevets

## EUROPEAN SEARCH REPORT

**Application Number**

EP 25 22 2654

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/087083 A1 (KONINKLIJKE PHILIPS NV [NL]) 17 May 2018 (2018-05-17) * page 10, line 7 - page 18, line 6; figure 5 * ----- | 1-8 | INV. A61B6/06 A61B6/00 |
| A | EP 3 387 997 A1 (SIEMENS HEALTHCARE GMBH [DE]) 17 October 2018 (2018-10-17) * paragraph [0025] - paragraph [0085] * ----- | 1-8 | |
| A | EP 4 212 101 A1 (SIEMENS HEALTHCARE GMBH [DE]; SIEMENS HEALTHINEERS AG [DE]) 19 July 2023 (2023-07-19) * paragraph [0044] - paragraph [0081]; figure 2 * ----- | 1-8 | |

**TECHNICAL FIELDS SEARCHED (IPC)**

A61B

The present search report has been drawn up for all claims

| Place of search | Date of completion of the search | Examiner |
|---|---|---|
| Munich | 4 May 2026 | Hooper, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 4 767 947 A1

## ANNEX TO THE EUROPEAN SEARCH REPORT
## ON EUROPEAN PATENT APPLICATION NO.

EP 25 22 2654

04-05-2026

| Patent document cited in search report | | | Publication date | Patent family member(s) | | | Publication date |
|---|---|---|---|---|---|---|---|
| WO 2018087083 | A1 | | 17-05-2018 | CN | 109937012 | A | 25-06-2019 |
| | | | | EP | 3537976 | A1 | 18-09-2019 |
| | | | | JP | 7138631 | B2 | 16-09-2022 |
| | | | | JP | 2020500579 | A | 16-01-2020 |
| | | | | RU | 2019118036 | A | 10-12-2020 |
| | | | | US | 2020058389 | A1 | 20-02-2020 |
| | | | | WO | 2018087083 | A1 | 17-05-2018 |
| EP 3387997 | A1 | | 17-10-2018 | CN | 108814634 | A | 16-11-2018 |
| | | | | EP | 3387997 | A1 | 17-10-2018 |
| | | | | US | 2018296177 | A1 | 18-10-2018 |
| | | | | US | 2018296178 | A1 | 18-10-2018 |
| EP 4212101 | A1 | | 19-07-2023 | CN | 116433574 | A | 14-07-2023 |
| | | | | EP | 4212101 | A1 | 19-07-2023 |
| | | | | US | 2023233169 | A1 | 27-07-2023 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240193779

- KR 102604560